# EUROPEAN PATENT SPECIFICATION

(11) **EP 2 561 862 B1**
(45) Date of publication and mention of the grant of the patent: **05.08.2015**
(21) Application number: 11762708.3
(22) Date of filing: 25.03.2011
(51) Int. Cl.: A61K 9/127, A61K 8/14, A61K 8/368, A61K 8/44, A61K 8/46, A61K 8/67, A61K 8/86, A61K 8/894, A61K 31/192, A61K 31/195, A61K 31/198, A61K 31/375, A61K 47/02, A61K 47/10, A61K 47/14, A61K 47/20, A61K 47/34, A61K 47/46, A61Q 19/00, A61Q 19/02

(54) **VESICLE-CONTAINING COMPOSITION**
VESIKELHALTIGE ZUSAMMENSETZUNG
COMPOSITION CONTENANT DES VÉSICULES

(30) Priority: 30.09.2010 JP 2010220684; 31.03.2010 JP 2010082301
(43) Date of publication of application: 27.02.2013
(73) Proprietor: SHISEIDO COMPANY, LTD., Tokyo 104-0061 (JP)
(72) Inventor: NISHIDA Miharu, Yokohama-shi Kanagawa 224-8558 (JP); WATANABE Kei, Yokohama-shi Kanagawa 224-8558 (JP); MATSUSHITA Yuji, Yokohama-shi Kanagawa 224-8558 (JP)
(74) Representative: Henkel, Breuer & Partner
(86) International application number: PCT/JP2011/057378
(87) International publication number: WO 2011/122477

(56) References cited:
- EP-A1- 2 149 362
- WO-A1-2004/050045
- WO-A1-2008/143140
- WO-A2-2005/102248
- WO-A2-2007/053424
- JP-A- 2003 160 476
- JP-A- 2007 031 336
- JP-A- 2008 024 681
- JP-A- 2008 255 109
- JP-A- 2009 242 326
- JP-A- 2009 249 360
- US-A1- 2009 171 012
- NEWTON J ET AL: "SILICONE-BASED VESICLE DELIVERY SYSTEMS", COSMETICS & TOILETRIES, WHEATON, IL, US, vol. 119, no. 12, 1 December 2004 (2004-12-01), pages 53-60, XP009085610, ISSN: 0361-4387

## Description

### RELATED APPLICATIONS

This application claims the priority of Japanese Patent Application No. 2010-82301 filed on March 31, 2010 and Japanese Patent Application No. 2010-220684 filed on September 30, 2010.

### FIELD OF THE INVENTION

The present invention relates to a vesicle-containing composition, and particularly relates to improvement of stability of vesicles if a water-soluble medicinal agent is contained even at a high concentration in the composition.

### BACKGROUND OF THE INVENTION

Of the amphipathic compounds having both hydrophilicity and hydrophobicity, compounds such as phospholipid are known to form a spherical endoplasmic reticulum composed of a bilayer membrane (lamellar layer) in an aqueous phase. Such a bilayer membrane endoplasmic reticulum, which is called as liposome or vesicle, is capable of stably holding an aqueous component in the interior of the endoplasmic reticulum or an oily component in the interior of the endoplasmic reticulum membrane. Because of this, if a vesicle is allowed to carry e.g., a medicinal agent and injected into a living body, efficacy of the medicinal agent can be maintained for a long time. Owing to the advantage, the vesicle is used as a microcapsule in the various fields including medicine, cosmetics and food. In addition, due to formation of a liposome or a vesicle, a composition having good appearance (transparency) and good sense of use can be obtained. Thus, it is expected that the vesicle may be used as a cosmetic base.

Recently, as an amphipathic compound capable of forming such a vesicle, a silicone-based surfactant has been known. It is easier to form a vesicle from a silicone-based surfactant than other surfactants, whereas, it is difficult to maintain stability against time and temperature and blend a fragrance component stably. In these respects, a silicone-based surfactant had a problem in putting it into practical use as cosmetics. However, recently, vesicle-containing compositions in which these problems are improved have been proposed. For example, Japanese Patent Application Laid-Open No. 2008-24681 discloses that sufficient stability for use as a cosmetic base can be given to a vesicle by the presence of a water-soluble low molecular weight surfactant (anionic surfactant) in the external phase of a vesicle formed of a silicone-based surfactant.

Furthermore, International Publication No. WO 2008/143140 discloses that a fragrance can be held within a bilayer membrane of a vesicle by mixing an oily component containing a silicone oil and a fragrance in advance, and blending the mixture with an ethanolic or similar solution of a silicone-based surfactant, thereby mixing with an aqueous formulation to form vesicles. Accordingly, the publication discloses a vesicle-containing composition comprising (A) a perfume, (B) a silicone oil, (C) a silicone surfactant, (D) one or more selected from the group consisting of ethanol, propylene glycol, dipropylene glycol, and 1,3-butylene glycol, and (E) water, wherein the silicone surfactant forms vesicles, and wherein the perfume and the silicone oil are present in the vesicle bilayer membrane.

As described above, a vesicle formed of a silicone-based surfactant has been aggressively investigated in order to improve its practical performance in cosmetics.

### DISCLOSURE OF THE INVENTION

### PROBLEM TO BE SOLVED BY THE INVENTION

In the meantime, in cosmetics, to give efficacy such as a whitening effect, a water-soluble medicinal agent such as tranexamic acid is often blended in an effective amount or more. However, in the aforementioned improved vesicle-containing composition with a silicone-based surfactant, when a water-soluble medicinal agent was blended in a certain amount or more with a water phase, vesicles were formed, but often damaged during storage and an oily content leaked out to yield white turbidity. Accordingly, it has been desired to develop a vesicle-containing composition capable of maintaining high stability even if a large amount of water-soluble medicinal agent is blended.

The present invention was made in view of problems of the conventional techniques and provides a vesicle-containing composition having an excellent stability in the presence of water-soluble medicinal agent.

### MEANS TO SOLVE THE PROBLEM

The present inventors conducted intensive studies with a view to solving the problems of conventional techniques. As a result, they found that a vesicle-containing composition having excellent stability can be obtained even if a formulation contains a water-soluble medicinal agent in a large amount by protecting vesicles formed of a silicone-based surfactant by blending a predetermined anionic surfactant. Based on the finding, the present invention was accomplished. The invention is as defined in the claims.

In one aspect, the invention thus relates to a vesicle-containing composition, comprising:
(A) a silicone-based surfactant,
(B) one or more anionic surfactants selected from polyoxyethylene alkyl (12 to 15) ether phosphate, acyl methyl taurate and acyl glutamate in an amount of 0.001 to 0.2 mass%,
(C) a polar oil having IOB of 0.05 to 0.80 and/or silicone oil, and
(D) water containing a water-soluble medicinal agent selected from tranexamic acid, tranexamic acid methyl amide hydrochloride, potassium 4-methoxysalicylate, ascorbic acid glucoside and ethyl vitamin C in an amount of 0.5 to 5 mass% based on the composition,
wherein the (A) silicone-based surfactant forms vesicles; the (B) anionic surfactant(s) attaches to a surface of the vesicles; and the (C) polar oil and/or silicone oil is present within a bilayer membrane of the vesicles,
wherein acyl methyl taurate is selected from coconut oil fatty acid methyl taurate, palm kernel oil fatty acid methyl taurate, hydrogenated palm kernel oil fatty acid methyl taurate, beef tallow fatty acid methyl taurate, hydrogenated beef tallow fatty acid methyl taurate, caproyl methyl taurate, lauroyl methyl taurate, myristoyl methyl taurate, palmitoyl methyl taurate, stearoyl methyl taurate, oleoyl methyl taurate and cocoyl methyl taurate,
wherein acyl glutamate is selected from stearoyl glutamate, cocoyl glutamate, coconut oil fatty acid glutamate, lauroyl glutamate, palm kernel oil fatty acid glutamate, hydrogenated palm kernel oil fatty acid glutamate, beef tallow fatty acid glutamate and hydrogenated beef tallow fatty acid glutamate, and
wherein a counter ion to the salts is selected from Na, K, triethanol amine and ammonia.

In the vesicle-containing composition, it is preferable that the (B) anionic surfactant(s) is one or more selected from polyoxyethylene alkyl (12 to 15) ether phosphate, stearoyl methyl taurate, cocoyl glutamate and stearoyl glutamate.

In the vesicle-containing composition, it is preferable that the (A) silicone-based surfactant is polyoxyalkylene-modified silicone represented by the following formula (1): 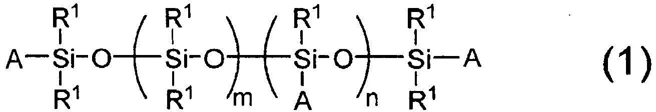 wherein R¹ represents hydrogen or an alkyl group having 1 to 6 carbon atoms; at least one of A is a polyoxyalkylene group represented by the formula: - (CH₂)ₐ- (C₂H₄O)_{b}- (C₃H₆O)_{c}-R² wherein R² represents hydrogen or an alkyl group having 1 to 6 carbon atoms, a represents an integer of 1 to 6, b represents an integer of 0 to 50, c represents an integer of 0 to 50 and b + c represents at least 5; the other(s) of A represents hydrogen or an alkyl group having 1 to 6 carbon atoms; m represents an integer of 1 to 200; and n represents an integer of 0 to 50.

In the vesicle-containing composition, it is preferable that a content of the (C) polar oil having IOB of 0.05 to 0.80 and/or silicone oil is 0.001 to 0.3 mass%.

In the vesicle-containing composition, it is preferable that a vesicle formed of the (A) silicone-based surfactant further contains, (E) sodium pyrosulfite in an amount of 0.001 to 0.05 mass% and/or dibutylhydroxytoluene in an amount of 0.005 to 0.05 mass%.

In the vesicle-containing composition, it is preferable that the (D) water containing a water-soluble medicinal agent in an amount of 0.5 to 5 mass% based on the composition further contains one or two or more selected from the group consisting of ethanol, propylene glycol, dipropylene glycol and 1,3-butylene glycol.

In another aspect, the invention relates to a method for producing a vesicle-containing composition, comprising:
mixing (A) a silicone-based surfactant and (C) a polar oil having IOB of 0.05 to 0.80 and/or silicone oil,
mixing with (D) water containing a water-soluble medicinal agent selected from tranexamic acid, tranexamic acid methyl amide hydrochloride, potassium 4-methoxysalicylate, ascorbic acid glucoside and ethyl vitamin C, and
adding and mixing (B) one or more anionic surfactants selected from polyoxyethylenealkyl (12 to 15) ether phosphate, acyl methyl taurate and acyl glutamate,
wherein acyl methyl taurate is selected from coconut oil fatty acid methyl taurate, palm kernel oil fatty acid methyl taurate, hydrogenated palm kernel oil fatty acid methyl taurate, beef tallow fatty acid methyl taurate, hydrogenated beef tallow fatty acid methyl taurate, caproyl methyl taurate, lauroyl methyl taurate, myristoyl methyl taurate, palmitoyl methyl taurate, stearoyl methyl taurate, oleoyl methyl taurate and cocoyl methyl taurate,
wherein acyl glutamate is selected from stearoyl glutamate, cocoyl glutamate, coconut oil fatty acid glutamate, lauroyl glutamate, palm kernel oil fatty acid glutamate, hydrogenated palm kernel oil fatty acid glutamate, beef tallow fatty acid glutamate and hydrogenated beef tallow fatty acid glutamate, and
wherein a counter ion to the salts is selected from Na, K, triethanol amine and ammonia.

In the method for producing a vesicle-containing composition, it is preferable to comprise:
mixing the (A) silicone-based surfactant and (C) a polar oil having IOB of 0.05 to 0.80 and/or silicone oil,
mixing with a mixture obtained by mixing (E) sodium pyrosulphite and/or dibutylhydroxytoluene and (D) water containing a water-soluble medicinal agent selected from tranexamic acid, tranexamic acid methyl amide hydrochloride, potassium 4-methoxysalicylate, ascorbic acid glucoside and ethyl vitamin C, and
adding and mixing one or more anionic surfactant (B) selected from polyoxyethylenealkyl (12 to 15) ether phosphate, acyl methyl taurate, and acyl glutamate,
wherein acyl methyl taurate is selected from coconut oil fatty acid methyl taurate, palm kernel oil fatty acid methyl taurate, hydrogenated palm kernel oil fatty acid methyl taurate, beef tallow fatty acid methyl taurate, hydrogenated beef tallow fatty acid methyl taurate, caproyl methyl taurate, lauroyl methyl taurate, myristoyl methyl taurate, palmitoyl methyl taurate, stearoyl methyl taurate, oleoyl methyl taurate and cocoyl methyl taurate,
wherein acyl glutamate is selected from stearoyl glutamate, cocoyl glutamate, coconut oil fatty acid glutamate, lauroyl glutamate, palm kernel oil fatty acid glutamate, hydrogenated palm kernel oil fatty acid glutamate, beef tallow fatty acid glutamate and hydrogenated beef tallow fatty acid glutamate, and
wherein a counter ion to the salts is selected from Na, K, triethanol amine and ammonia.

### EFFECT OF THE INVENTION

According to the present invention, a vesicle-containing composition having extremely excellent stability can be obtained even in the presence of a water-soluble medicinal agent. Owing to this, various types of water-soluble medicinal agents can be blended in cosmetics using vesicles formed of a silicone-based surfactant in a sufficient amount to add a further function.

### DESCRIPTION OF THE PREFERRED EMBODIMENT

Now, preferable embodiments of the present invention will be described below.

The vesicle-containing composition according to the present invention comprises (A) a silicone-based surfactant, (B) one or more anionic surfactants selected from a polyoxyethylenealkyl (12 to 15) ether phosphate, acyl methyl taurate and acyl glutamate in an amount of 0.001 to 0.2 mass%, (C) a polar oil having IOB of 0.05 to 0.80 and/or silicone oil, (D) water containing a water-soluble medicinal agent in an amount of 0.5 to 5 mass% based on the composition, in which the (A) silicone-based surfactant forms vesicles; the (B) anionic surfactant(s) attaches to the surface of the vesicles; and the (C) polar oil and/or silicone oil is present within the bilayer membrane of the vesicles. (A) Silicone-based surfactant

The (A) silicone-based surfactant to be used in the present invention is not particularly limited as long as it is a surfactant having a polysiloxane structure as a hydrophobic group. Specific examples of the (A) silicone-based surfactant include polyoxyalkylene-modified silicone represented by the following formula (1): 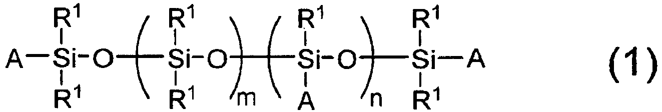 wherein R¹ represents hydrogen or an alkyl group having 1 to 6 carbon atoms; at least one of A is a polyoxyalkylene group represented by the formula: - (CH₂)ₐ- (C₂H₄O)_{b}- (C₃H₆O)_{c}-R² wherein R² represents hydrogen or an alkyl group having 1 to 6 carbon atoms, a represents an integer of 1 to 6, b represents an integer of 0 to 50, c represents an integer of 0 to 50 and b + c represents at least 5; the other(s) of A represents hydrogen or an alkyl group having 1 to 6 carbon atoms; m represents an integer of 1 to 200 and n represents an integer of 0 to 50.

Herein, in the formula (1), R¹, which corresponds to a side chain relative to a main-chain of a polysiloxane structure, represents hydrogen or an alkyl group having 1 to 6 carbon atoms. Furthermore, these may be the same or different. To explain more specifically, if all R¹ are methyl groups, the formula represents a dimethylpolysiloxane structure; and if R¹ are a methyl group and a phenyl group, the formula represents a methylphenylpolysiloxane structure. Furthermore, in the main chain polysiloxane structure, A is the site to which a polyoxyalkylene group is to be introduced. At least one of A is a polyoxyalkylene group represented by the formula: - (CH₂)ₐ- (C₂H₄O)_{b}- (C₃H₆O)_{c}-R² wherein R² represents hydrogen or an alkyl group having 1 to 6 carbon atoms; a represents an integer of 1 to 6, b represents an integer of 0 to 50, c represents an integer of 0 to 50 and b + c represents at least 5.

Note that, in the formula (1), in the case where a part of A is the polyoxyalkylene group, the other part of A may be hydrogen or an alkyl group having 1 to 6 carbon atoms. To describe more specifically, if both ends A are polyoxyalkylene groups, the formula A-B-A represents polyoxyalkylene-modified silicone. In another case, if a polyoxyalkylene group is present at a position except both ends, the formula represents a pendant-form polyoxyalkylene-modified silicone. The polyoxyalkylene group may be any of a polyoxyethylene group, a polyoxypropylene group and a polyoxyethylene-polyoxypropylene group. The mole number of an unsubstituted polysiloxane structure represented by m is 1 to 200. The mole number of a polyoxyalkylene substituted polysiloxane structure represented by n is 0 to 50. Note that, in the case of n = 0, either one or both of the two ends A must be a polyoxyalkylene group(s).

Further specific examples of the (A) silicone-based surfactant to be used in the present invention include polyoxyethylene (12 mol)-modified dimethylpolysiloxane (pendant type polyoxyalkylene-modified silicone obtained by substituting the side chain methyl group of a linear dimethylpolysiloxane with a polyoxyethylene (12 mol) group), polyoxyethylene (8 mol)-modified dimethylpolysiloxane and polyoxyethylene (20 mol)-modified dimethylpolysiloxane. Other than these, an ABA type polyoxyethylene-methylsiloxane-polyoxyethylene block-copolymer, etc. are mentioned. In the case of these polyoxyethylene-modified silicones, the ratio of the molecular weight of the ethylene oxide occupying in the total molecular weight is desirably 20 to 60%. Note that, the (A) silicone-based surfactant to be used in the present invention can be also produced by a known method or a commercially available product may be used. Examples of the commercially available silicone-based surfactant include SH3772M, SH3773M, SH3775M (all manufactured by Dow Corning Silicone) and IM-22 (manufactured by Wacker Chemical). Furthermore, these (A) silicone-based surfactants may be used alone or in combination.

The (A) silicone-based surfactant is a component constituting a vesicle. In the vesicle-containing composition according to the present invention, the (A) silicone-based surfactant is contained in the form of vesicle. A vesicle can be easily formed by a known method as described later as the production method of the present invention. For example, in an aqueous formulation, the (A) silicone-based surfactant and, if necessary, one or more selected from ethanol, propylene glycol, dipropylene glycol and 1,3-butylene glycol are mixed and stirred to obtain vesicles formed of the (A) silicone-based surfactant in the aqueous formulation. Note that, the vesicle particle-size is not particularly limited; however, it is usually about 20 to 500 nm and preferably 50 to 200 nm.

The HLB of the (A) silicone-based surfactant is not particularly limited; however, in view of vesicle formation, HLB is preferably 4 to 12 and further preferably 6 to 9. If the HLB of the (C) silicone-based surfactant is outside the above range, it becomes difficult to form stable vesicles, and sometimes, an oily content containing the (C) polar oil cannot be efficiently taken into a vesicle bilayer membrane.

The content of the (A) silicone-based surfactant is not particularly limited as long as it is a content enough to form a vesicle; however, the content is preferably 0.1 to 10 mass% based on the total amount of composition and further preferably 0.2 to 5.0 mass%. If the content of the (A) silicone-based surfactant is low, the effect brought by vesicle formation cannot be obtained in some cases. In contrast, if the content is excessively large, stability of a vesicle deteriorates in some cases.

### (B) One or more anionic surfactants selected from polyoxyethylene alkyl (12 to 15) ether phosphate, acyl methyl taurate and acyl glutamate

The anionic surfactant to be used in the present invention is preferably a polyoxyethylenealkyl (12 to 15) ether phosphate represented by the following formula (2), acyl methyl taurate and/or acyl glutamate.

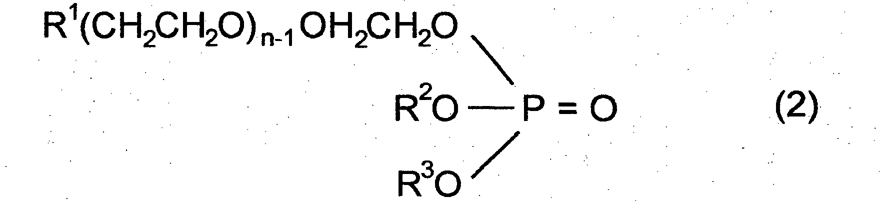

In the formula (2), R¹ represents an alkyl group having 12 to 15 carbon atoms. Furthermore, R² and R³ each independently represent R⁴O(CH₂CH₂O)ₘ₋₁OH₂CH₂- or a hydrogen group. R⁴ is an alkyl group having 12 to 15 carbon atoms. The addition mole number of oxyethylene represented by the sum of m and n is an integer of 1 to 30.

Such polyoxyethylenealkyl (12 to 15) ether phosphate includes, for example, polyoxyethylene (10 mol) lauryl ether, polyoxyethylene (3 mol) myristyl ether, polyoxyethylene (7 mol) dodecyl ether. As a commercially available product thereof, DDP-2 and NIKKOL TDP-10 (manufactured by Nikko Chemicals Co., Ltd.) can be preferably used.

The acyl methyl taurate is selected from coconut oil fatty acid methyl taurate, palm kernel oil fatty acid methyl taurate, hydrogenated palm kernel oil fatty acid methyl taurate, beef tallow fatty acid methyl taurate, hydrogenated beef tallow fatty acid methyl taurate, caproyl methyl taurate, lauroyl methyl taurate, myristoyl methyl taurate, palmitoyl methyl taurate, stearoyl methyl taurate, oleoyl methyl taurate and cocoyl methyl taurate.

The acyl glutamate is selected from stearoyl glutamate, cocoyl glutamate, coconut oil fatty acid glutamate, lauroyl glutamate, coconut oil fatty acid glutamate, palm kernel oil fatty acid glutamate, hydrogenated palm kernel oil fatty acid glutamate, beef tallow fatty acid glutamate, and hydrogenated beef tallow fatty acid glutamate.

A counter ion to the salts is selected from Na, K, triethanol amine and ammonia.

In the present invention, as the component (B), particularly, one or more elements selected from polyoxyethylenealkyl (12 to 15) ether phosphate, stearoyl methyl taurate, cocoyl glutamate and stearoyl glutamate are preferably used. As a commercially available product thereof, NIKKOL SMT (manufactured by Nikko Chemicals Co., Ltd.), Amisoft CK-22 and Amisoft HS-11P (F) (manufactured by Ajinomoto Co., Inc.) can be preferably used.

It is conceivable that the (B) anionic surfactant(s) attaches to the surface of a vesicle formed of the (A) silicone-based surfactant and protects the vesicle from damage caused by blending of a medicinal agent. Therefore, the (B) anionic surfactant(s) is added to the system after vesicles are formed of the (A) silicone-based surfactant in the (D) water containing a water-soluble medicinal agent. Owing to this, the (B) anionic surfactant(s) contributes to protection and stabilization of the outer surface of the vesicles. In the case where the (B) anionic surfactant(s) is added during the vesicle formation, the protective effect of the anionic surfactant(s) on vesicles sometimes becomes insufficient.

The content of the (B) anionic surfactant(s) is such that vesicles formed of a silicone-based surfactant can be protected, namely 0.001 to 0.2 mass% based on the composition, and preferably 0.005 to 0.1 mass%. If the content of the anionic surfactant(s) is less than 0.001, stability of the composition is sometimes insufficient; whereas if the content exceeds 0.2 mass%, the content may have an adverse effect on the stability of vesicles.

### (C) Polar oil having IOB of 0.05 to 0.80 and/or silicone oil

In the present invention, as the oily component, a polar oil having IOB of 0.05 to 0.80 and/or silicone oil is blended. If a polar oil having an IOB value beyond 0.80 is used alone without blending the oily component, the stability of vesicles becomes insufficient.

The polar oil having IOB of 0.05 to 0.80 is not particularly limited as long as it is an oily component preferably having an IOB value within the range. Note that, the IOB value of the oily component can be calculated by a known calculation method based on the structure.

Such polar oil includes, for example, isostearic acid, isopropyl myristate, cetyl octanoate, octyldodecyl myristate, isopropyl palmitate, butyl stearate, hexyl laurate, myristyl myristate, decyl oleate, hexyldecyl dimethyloctanoate, isononyl isononanoate, cetyl lactate, myristyl lactate, acetylated lanolin, isocetyl stearate, isocetyl isostearate, cetyl ethylhexanoate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glyceryl di-2-heptylundecanoate, trimethylolpropane tri-2-ethylhexanoate, trimethylolpropane triisostearate, pentaerythrityl tetra-2-ethylhexanoate, glyceryl tri-2-ethylhexanoate (triethylhexanoin), trimethylolpropane triisostearate, cetylisooctanoate, cetyl 2-ethylhexanotate, 2-ethylhexyl palmitate, alkyl (C12 to 15) benzoate, cetearyl isononanoate, glyceryl tri(caprylate/caprate), butylene glycol (dicaprylate/caprate), glyceryl trimyristate, glyceride tri-2-heptylundecanoate, castor oil fatty acid methyl ester, oleyl oleate, cetostearyl alcohol, acetoglyceride, 2-heptylundecyl pahnitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hyxyldecyl palmitate, 2-hexyldecyl adipate, diisopropyl sebacate, di-2-ethylhexyl succinate, ethyl acetate, butyl acetate, amyl acetate, triethyl citrate, 2-ethylhexyl p-methoxycinnamate, tripropylene glycol dipivalate, and 2-ethylhexyl 2-cyano-3,3-diphenylacrylate. (C) polar oil can be used alone or in combination by arbitrarily selecting two or more.

The silicone oil to be used in the present invention is not particularly limited as long as it is an oily component having a polysiloxane structure, and oily components having either a linear structure or a cyclic structure and either being volatile or non-volatile may be used. Specific examples of the silicone oil include chain-form silicones such as dimethylpolysiloxane, methylphenylpolysiloxane and methylhydrogenpolysiloxane; and cyclic silicones such as octamethylcyclotetrasiloxane, decamethylcyclopentasiloxane and dodecamethylcyclohexasiloxane. These can be used alone or in combination by arbitrarily selecting two or more.

Note that, of these silicone oils, a volatile cyclic silicone oil, particularly octamethylcyclotetrasiloxane and decamethylcyclopentasiloxane can be preferably used. If these volatile cyclic silicone oils are used, a fragrance component can be incorporated into a vesicle bilayer membrane in a larger amount and, in addition, if a vesicle-containing composition is used as an external preparation, excellent sense of use including low stickiness can be obtained.

Furthermore, as a non-volatile silicone oil, particularly, a low-viscosity silicone oil such as diphenylsiloxyphenyltrimethicone and methylpolysiloxane is preferably used.

The content of the (C) polar oil and/or silicone oil is preferably 0.001 to 0.3 mass% in total based on the total amount of composition. Furthermore, in the vesicle-containing composition of the present invention, as long as the (C) polar oil and/or silicone oil is blended a polar oil having IOB outside the range of 0.05 to 0.80 and a non-polar oil such as a mineral oil may be used in combination.

### (D) Water containing a water-soluble medicinal agent in an amount of 0.5 to 5 mass% based on the composition

### Water-soluble medicinal agent

With the composition of the present invention, water containing a water-soluble medicinal agent is blended. Vesicles formed of silicone-based surfactant decrease in stability by the presence of a water-soluble medicinal agent of usually, 0.5 mass% or more; however, in the present invention, since vesicles are protected with the (B) anionic surfactant(s), high stability can be maintained even in the presence of a water-soluble medicinal agent.

In the vesicle-containing composition according to the present invention, the content of a water-soluble medicinal agent is 0.5 to 5 mass% based on the total amount of composition.

For example, in an attempt of blend a water-soluble medicinal agent in order to give an additional effect to a product, if the upper limit of blending is less than 0.5 mass%, the effective amount of medicinal agent cannot be blended. Furthermore, since a medicinal agent cannot be introduced in a vesicle internal phase in a concentration of 0.5% or more, in a conventional vesicle-containing composition, vesicles are cannot be sufficiently used as microcapsules. Even in this case, if the vesicle-containing composition of the present invention is used, a water-soluble medicinal agent can be blended in a wide concentration range while maintaining stability of vesicles.

A water-soluble medicinal agent used in the present invention is applicable for drugs, quasi-drugs or cosmetics, and is selected from tranexamic acid, tranexamic acid methyl amide hydrochloride, potassium 4-methoxysalicylate, ascorbic acid glucoside and ethyl vitamin C.

### Water

The content of water is not particularly limited; however, the content is preferably 70 to 95 mass% based on the total amount of composition, and further preferably 80 to 90 mass%. If the content of water is low, vesicles sometimes cannot be formed.

To water, not only the water-soluble medicinal agent but also an arbitrary aqueous component usually used in pharmaceutical products, cosmetics etc. may be blended within the range of the content which does not affect stability of vesicles.

Particularly, as the aqueous component, in view of vesicle stability and sense of use, one or two or more components selected from ethanol and polyol are preferably blended.

The polyol includes, for example, ethylene glycol, propylene glycol, 1,3-butylene glycol, tetramethylene glycol, glycerin, sorbitol, diethylene glycol, dipropylene glycol, tetramethylene glycol, diglycerin, polyethylene glycol and polypropylene glycol, and propylene glycol, dipropylene glycol and 1,3-butylene glycol are preferable. Also, one or more aqueous components selected from ethanol and polyol can be blended in an amount of 1 to 20 mass% and preferably 3 to 10 mass% based on the total amount of the composition.

### (E) Sodium pyrosulphite and/or dibutylhydroxytoluene

Like the vesicle-containing composition according to the present invention, if vesicles are produced by using a silicone-based surfactant, odor presumably derived from the silicone-based surfactant often generates. Such odor can be suppressed by blending sodium pyrosulphite in an amount of 0.001 to 0.05 mass% and preferably 0.003 to 0.03 mass% based on the vesicle-containing composition and/or blending dibutylhydroxytoluene in an amount of 0.005 to 0.05 mass% and preferably 0.01 to 0.03 mass%.

In the case of less than the content, an odor suppressing effect cannot be sufficiently exerted. In contrast, in the case of beyond the content, odor derived from sodium pyrosulfite may generate or discolor due to dibutylhydroxytoluene may occur.

Sodium pyrosulphite and/or dibutylhydroxytoluene exerts an effect of suppressing odor of a silicone-based surfactant in the present invention.

The effect is a significant effect found, for the first time, by the present inventors. Sodium pyrosulphite and/or dibutylhydroxytoluene serves as an odor suppressing agent for suppressing odor derived from a silicone-based surfactant in a vesicle-containing composition formed by using a silicone-based surfactant.

The vesicle-containing composition according to the present invention can be produced by using the essential components (A) to (D). Note that, a known method can be applied to a production method for a vesicle-containing composition. For example, the composition of the present invention can be obtained by mixing (A) the silicone-based surfactant and (C) an oily component containing the polar oil having IOB of 0.05 to 0.80 and/or the silicone oil; mixing this with (D) water containing the water-soluble medicinal agent and optionally the arbitrary aqueous component, and further adding and mixing (B) the predetermined anionic surfactant.

In the production method, vesicles are formed by mixing an aqueous formulation and a silicone-based surfactant. Herein, the aqueous formulation refers to a mixture of (D) water containing the water-soluble medicinal agent to which the arbitrary aqueous component is optionally added.

If the aqueous formulation and a silicone-based surfactant are mixed to obtain vesicles formed of the silicone-based surfactant in the aqueous phase and an oily component containing polar oil having IOB of 0.05 to 0.80 and/or silicone oil which has been mixed with the silicone-based surfactant is taken into a vesicle bilayer membrane. To the vesicle-containing aqueous solution thus obtained, if a predetermined anionic surfactant is added, the anionic surfactant forms micelle around the outer periphery of a vesicle bilayer membrane. The micelle that is present around (attached to) the outer periphery of a vesicle is considered to greatly contribute to stability of vesicle membrane. Note that, since a vesicle is formed in advance in the step, the anionic surfactant cannot enter the internal phase of the vesicle and thus the micelle formed of the anionic surfactant substantially comes to be present only in the external phase of the vesicle.

As mentioned above, to the vesicle-containing aqueous solution formed by mixing an aqueous formulation and a silicone-based surfactant in advance, a predetermined anionic surfactant is added. In this manner, a micelle formed of the anionic surfactant comes to be substantially present only in the external phase of the vesicle. As a result, the stability of the vesicle is improved. Note that, for example, if a silicone-based surfactant and a predetermined anionic surfactant are simultaneously added to and mixed with an aqueous formulation, the concomitantly blended anionic surfactant directly affect the ordered structure of a vesicle bilayer membrane that is to be formed of the silicone-based surfactant, with the result that a vesicle may not be formed. Furthermore, even if a vesicle can be formed, the anionic surfactant comes to be present in both the internal phase and external phase of the vesicle, with the result that a desired effect of stabilizing a vesicle may not be obtained.

Note that, in the present invention, in the case where (E) sodium pyrosulphite and/or dibutylhydroxytoluene is blended as an agent for suppressing odor derived from a silicone-based surfactant, for example, the following production method may be mentioned. The vesicle-containing composition of the present invention can be obtained by mixing and dissolving (A) the silicone-based surfactant and (C) an oily component containing the polar oil having IOB of 0.05 to 0.80 and/or the silicone oil to ethanol or a humectant, mixing with the mixture obtained by mixing a predetermined amount of (E) sodium pyrosulphite and/or dibutylhydroxytoluene and (D) water containing the water-soluble medicinal agent and optionally the arbitrary aqueous component, and adding and mixing (B) the predetermined anionic surfactant.

The vesicle-containing composition according to the present invention can be preferably used, for example, as cosmetics. If the composition is used as cosmetics, not only the components but also other components usually used in cosmetics, pharmaceutical products and quasi drugs can be appropriately blended, within the contents which do not affect vesicle formation and its stability. Note that, the other formulation components may be appropriately blended in an oily component mixture or in an aqueous formulation before and after vesicle formation. Furthermore, for example, if an arbitrary medicinal-agent component is blended with an aqueous formulation to form vesicles and thereafter the external phase is replaced, a microcapsule composition having the medicinal-agent component within the internal phase alone can be prepared.

Use of the cosmetics according to the present invention is not particularly limited; however, preferable examples of use include lotion, beauty lotion and hair liquid.

### EXAMPLES

The present invention will be more specifically described below by way of Examples of the present invention; however, the present invention is not limited to these. Note that, unless otherwise specified, the content is expressed by mass%.

The vesicle-containing compositions obtained in accordance with the formulation and production method shown in the following Table 1 were evaluated for appearance stability. The evaluation method for appearance stability is as follows.

### Measurement of turbidity change

Each of the test samples obtained after storage of the prepared vesicles at 50°C and -10°C for a month was subjected to measurement of absorbance (Abs.) at a wavelength of 600 nm by an absorptiometer (Ubest-55 manufactured by JASCO Corporation) and a turbidity change (%)(Abs. after storage/ Abs. immediately after preparation) was calculated and appearance stability was evaluated based on the following evaluation criteria.

### (Evaluation criteria for appearance stability)

⊚: Turbidity change is 5% or less
○: Turbidity change is less than 10%
Δ: Turbidity change is 10 to 20%
×: Turbidity change is 20% or more

**Table 1**

| | | **Experimental Example 1-1** | **Experimental Example 1-2** | **Experimental Example 1-3** | **Experimental Example 1-4** |
|---|---|---|---|---|---|
| (1) **Purified water** | | **Balance** | **Balance** | **Balance** | **Balance** |
| (2) **Ethanol** | | 5 | 5 | 5 | 5 |
| (3) **Glycerin** | | 3 | 3 | 3 | 3 |
| (4) **1,3-butvlene glycol** | | 5 | 5 | 5 | 5 |
| (5) POE(12)dimethylpolysiloxane (*1) | | 0.5 | 0.5 | 0.5 | 0.5 |
| (6) Sodium stearoyl methyl taurate | | 0.02 | 0.02 | - | - |
| (7) POE (2) sodium lauryl ether carboxylate | | - | - | 0.02 | 0.02 |
| (8) **Cetyl ethylhexanoate (*2)** | | 0.05 | 0.05 | 0.05 | 0.05 |
| (9) **Tranexamic acid** | | 1 | 0.5 | 0.5 | - |
| (10) Citric acid | | 0.03 | 0.03 | 0.03 | 0.03 |
| (11) Sodium citrate | | 0.07 | 0.07 | 0.07 | 0.07 |
| (12)EDTA-2Na·2H₂O | | 0.03 | 0.03 | 0. 03 | 0.03 |
| (13) **Phenoxyethanol** | | 0.5 | 0.5 | 0.5 | 0.5 |
| (14) **Fragrance** | | **Appropriate amount** | **Appropriate amount** | **Appropriate amount** | **Appropriate amount** |
| **Appearance stability** | 50°C | ⊚ | ⊚ | Δ | ⊚ |
| | -10°C | ⊚ | ⊚ | Δ | ⊚ |

| | | | | | |
|---|---|---|---|---|---|
| (*1) SH3773M manufactured by Dow Coming Toray Co., Ltd. (*2) CIO manufactured by Nihon Surfactant Kogyo K.K. | | | | | |

### (Experimental Examples 1-3 and 1-4 are not according to the invention)

### (Production method)

To an aqueous phase, which was prepared by mixing and dissolving components (3), (4) and (9) to (12) with component (1), a mixture, which was prepared by mixing and dissolving components (5), (8), (13) and (14) with component (2), was added. To the mixture, component (6) or (7) was added, mixed and dissolved to obtain a vesicle-containing composition.

As shown in Table 1, vesicle-containing compositions of Experimental Examples 1-1 and 1-2 containing a predetermined anionic surfactant (sodium stearoyl methyl taurate) all showed excellent stability even in the presence of 0.5 mass% or more of a water-soluble medicinal agent (tranexamic acid) at either one of storage temperatures of 50°C and -10°C.

In contrast, vesicle-containing compositions of Experimental Examples 1-3 and 1-4 containing another anionic surfactant (POE (2) sodium lauryl ether carboxylate), stability was maintained in the absence of the water-soluble medicinal agent (Experimental Example 1-3); however, white turbidity yielded in the presence of 0.5 mass% of a water-soluble medicinal agent (tranexamic acid) during storage at 50°C and -10°C and thus stability was poor (Experimental Example 1-4).

From the above results, in the present invention, it is possible to maintain stability of vesicles by blending a predetermined anionic surfactant even if a water-soluble medicinal agent of 0.5 mass% or more was blended.

Furthermore, as a result of further experiments, it was found that in the present invention, a water-soluble medicinal agent can be blended in an amount of 0.5 to 5 mass% based on the total amount of composition.

The vesicle-containing compositions obtained in accordance with the formulation and production method shown in the following Table 2 were evaluated for appearance stability. The evaluation method for appearance stability is as above.

**Table 2**

| | | **Experimental Example 2-1** | **Experimental Example 2-2** | **Experimental Example 2-3** | **Experimental Example 2-4** | **Experimental Example 2-5** | **Experimental Example 2-6** |
|---|---|---|---|---|---|---|---|
| (1) **Purified water** | | **Balance** | **Balance** | **Balance** | **Balance** | **Balance** | **Balance** |
| (2) **Ethanol** | | 5 | 5 | 5 | 5 | 5 | 5 |
| (3) **Glycerin** | | 3 | 3 | 3 | 3 | 3 | 3 |
| (4) **1,3-butylene glycol** | | 5 | 5 | 5 | 5 | 5 | 5 |
| (5) POE(12)dimethylpolysiloxane (*1) | | 0.5 | 0.5 | 0.5 | 0.5 | 0.5 | 0.5 |
| (6) Sodium stearoyl methyl taurate | | 0.0005 | 0.001 | 0.005 | 0.1 | 0.2 | 0.3 |
| (7) **Triethylhexanoin (*2)** | | 0.05 | 0.05 | 0.05 | 0.05 | 0.05 | 0.05 |
| (8) **Tranexamic acid** | | 1 | 1 | 1 | 1 | 1 | 1 |
| (9) **Citric acid** | | 0.03 | 0.03 | 0.03 | 0.03 | 0.03 | 0.03 |
| (10) **Sodium citrate** | | 0.07 | 0.07 | 0.07 | 0.07 | 0.07 | 0.07 |
| (11)EDTA-2Na·2H₂O | | 0.03 | 0.03 | 0.03 | 0.03 | 0.03 | 0.03 |
| (12) **Phenoxyethanol** | | 0. 5 | 0.5 | 0.5 | 0.5 | 0.5 | 0.5 |
| (13) **Fragrance** | | **Appropriate amount** | **Appropriate amount** | **Appropriate amount** | **Appropriate amount** | **Appropriate amount** | **Appropriate amount** |
| **Appearance stability** | 50°C | Δ | ○ | ⊚ | ⊚ | ○ | Δ |
| | -10 °C | Δ | ○ | ⊚ | ⊚ | ○ | Δ |

| | | | | | | | |
|---|---|---|---|---|---|---|---|
| (*1) SH3773M manufactured by Dow Corning Toray Co., Ltd. (*2) RA-G-308 manufactured by Nippon Fine Chemical Co., Ltd. | | | | | | | |

### (Experimental Examples 2-1 and 2-6 are not according to the invention)

### (Production method)

To an aqueous phase, which was prepared by mixing and dissolving components (3), (4) and (8) to (11) with component (1), a mixture, which was prepared by mixing and dissolving components (5), (7), (12) and (13) with component (2), was added. To the mixture, component (6) was added, mixed and dissolved to obtain a vesicle-containing composition.

As shown in Table 2, vesicle-containing compositions of Experimental Examples 2-2 to 2-5, in which a predetermined anionic surfactant (sodium methyl stearoyl taurate) was blended in an amount of 0.001 to 0.2 mass%, showed excellent stability at either one of storage temperatures of 50°C and -10°C. In particular, Experimental Examples 2-3 and 2-4, in which the content of the predetermined anionic surfactant was 0.005 mass% and 0.1 mass%, respectively, had particularly high stability.

In contrast, in the vesicle-containing compositions of Experimental Examples 2-1 and 2-6, in which the content of a predetermined anionic surfactant was 0.0005 mass% and 0.3 mass%, respectively, sufficient stability was not obtained.

From the above results, in the present invention, the content of a predetermined anionic surfactant is 0.001 to 0.2 mass%, and preferably 0.005 to 0.1 mass%.

The vesicle-containing compositions obtained in accordance with the formulation and production method shown in the following Table 3 were evaluated for appearance stability. The evaluation method for appearance stability is as above.

**Table 3**

| | | **Experimental Example** 3-1 | **Experimental Example** 3-2 | **Experimental Example** 3-3 | **Experimental Example** 3-4 | **Experimental Example** 3-5 | **Experimental Example 3-6** | **Experimental Example 3-7** | **Experimental Example** 3-8 | **Experimental Example** 3-9 |
|---|---|---|---|---|---|---|---|---|---|---|
| (1) Purified water | | **Balance** | **Balance** | **Balance** | **Balance** | **Balance** | **Balance** | **Balance** | **Balance** | **Balance** |
| (2) Ethanol | | 5 | 5 | 5 | 5 | 5 | 5 | 5 | 5 | 5 |
| (3) Glycerin | | 3 | 3 | 3 | 3 | 3 | 3 | 3 | 3 | 3 |
| (4) 1,3-butylene glycol | | 5 | 5 | 5 | 5 | 5 | 5 | 5 | 5 | 5 |
| (5) POE(12)dimethylpolysiloxane (*1) | | 0.5 | 0.5 | 0.5 | 0.5 | 0.5 | 0.5 | 0.5 | 0.5 | 0.5 |
| (6) Sodium stearoyl methyl taurate | | 0.1 | 0.1 | 0.1 | 0.1 | 0.1 | 0.1 | 0.1 | 0.1 | 0.1 |
| (7) Pentaerythrityl tetraethylhexanoate (*2) (IOB =0.35) | | 0.0005 | 0.001 | 0.3 | 0.4 | ·· | ·· | ·· | 0.05 | 0.05 |
| (8) Octamethylcyclotetrasiloxane (Silicone oil) | | ·· | ·· | ·· | ·· | 0.05 | ·· | ·· | ·· | ·· |
| (9) Diethoxyethyl succinate (IOB = 1.0) | | ·· | ·· | ·· | ·· | ·· | 0.05 | ·· | 0.05 | ·· |
| (10) Liquid paraffin | | ·· | ·· | ·· | ·· | ·· | ·· | 0.05 | ·· | 0.05 |
| (11) Tranexamic acid | | 1 | 1 | 1 | 1 | 1 | 1 | 1 | 1 | 1 |
| (12) Citric acid | | 0.03 | 0.03 | 0.03 | 0.03 | 0.03 | 0.03 | 0.03 | 0.03 | 0.03 |
| (13) Sodium citrate | | 0.07 | 0.07 | 0.07 | 0.07 | 0.07 | 0.07 | 0.07 | 0.07 | 0.07 |
| (14)EDTA-2Na·2 H₂O | | 0.03 | 0.03 | 0.03 | 0.03 | 0.03 | 0.03 | 0.03 | 0.03 | 0.03 |
| (15) Phenoxyethanol | | 0.5 | 0.5 | 0.5 | 0.5 | 0.5 | 0.5 | 0.5 | 0.5 | 0.5 |
| (16) Fragrance | | Appropriate amount | Appropriate amount | Appropriate amount | Appropriate amount | Appropriate amount | Appropriate amount | Appropriate amount | Appropriate amount | Appropriate amount |
| Appearance stability | 50°C | ○ | ⊚ | ⊚ | ○ | ⊚ | × | × | ○ | ○ |
| | -10°C | ○ | ⊚ | ⊚ | ○ | ⊚ | × | × | ○ | ○ |

| | | | | | | | | | | |
|---|---|---|---|---|---|---|---|---|---|---|
| (*1) SH3773M manufactured by Dow Corning Toray Co., Ltd. (*2) RA-PE-408 manufactured by Nippon Fine Chemical Co., Ltd. | | | | | | | | | | |

### (Experimental Examples 3-6 and 3-7 are not according to the invention)

### (Production method)

To an aqueous phase, which was prepared by mixing and dissolving components (3), (4) and (11) to (14) with component (1), a mixture, which was prepared by mixing and dissolving components (5), (7) to (10), (15) and (16) with component (2), was added. To the mixture, component (6) was added, mixed and dissolved to obtain a vesicle-containing composition.

As shown in Table 3, vesicle-containing compositions of Experimental Examples 3-2 and 3-3, in which a polar oil (pentaerythrityl tetraethylhexanoate) having IOB of 0.35 was blended in an amount of 0.001 to 0.3 mass%, showed excellent stability.

In Experimental Examples 3-1 and 3-4, in which a polar oil having IOB of 0.35 was blended in an amount of 0.0005 mass% and 0.4 mass%, respectively, vesicle stability was satisfactory although it slightly decreased compared to the ones.

Furthermore, in Experimental Example 3-5, in which a silicone oil (octamethylcyclotetrasiloxane) was blended in place of the polar oil having IOB of 0.35, the stability was excellent.

In contrast, in Experimental Examples 3-6 and 3-7, in which diethoxyethyl succinate or nonpolar oil (liquid paraffin) was blended in place of the polar oil having IOB of 0.35, vesicle stability was extremely poor.

However, the vesicle-containing compositions of Experimental Examples 3-8 and 3-9, in which diethoxyethyl succinate or liquid paraffin was used in combination with the polar oil having IOB of 0.35, had high stability.

From the above results, in the present invention, the polar oil having IOB of 0.80 or less is preferably blended in an amount of 0.001 to 0.3 mass% particularly based on a composition. Furthermore, silicone oil is applicable similarly to the polar oil.

As a result of further experiments, in the present invention, it was found that a polar oil having IOB of 0.05 to 0.80 can be used.

Now, formulation examples of the vesicle-containing composition according to the present invention will be described below; however, these should not be construed as limiting the present invention. Each formulation was evaluated in accordance with the evaluation method and evaluation criteria.

### Formulation Example 1 Lotion

| (Component) | | (mass%) |
|---|---|---|
| (1) | Purified water | Balance |
| (2) | Ethanol | 3 |
| (3) | Glycerin | 8 |
| (4) | Dipropylene glycol | 7 |
| (5) | POE (12) dimethylpolysiloxane (SH3773M, manufactured by Dow Coming Silicone) | 1 |
| (6) | Polyoxyethylene alkyl (12-15) ether phosphate (2E.O.) (DDP-2, manufactured by Nikko Chemicals Co., Ltd.) | 0.05 |
| (7) | Diethylhexyl succinate (manufactured by Croda Japan) | 0.05 |
| (8) | Potassium 4-methoxysalicylate | 1 |
| (9) | Citric acid | 0.03 |
| (10) | Sodium citrate | 0.07 |
| (11) | EDTA-2Na.2H₂O | 0.03 |
| (12) | Phenoxyethanol | 0.5 |
| (13) | Fragrance | Appropriate amount |

### (Production method)

To a water phase prepared by mixing components (3), (4) and (8) to (11) with component (1), a mixture prepared by mixing and dissolving components (5), (7), (12) and (13) to component (2) and finally component (6) was added, mixed and dissolved to obtain a lotion.

The obtained lotion showed a turbidity change of 5% at 50°C and -10°C and showed excellent appearance stability (⊚).

### Formulation Example 2 Lotion

| (Component) | | (mass%) |
|---|---|---|
| (1) | Purified water | Balance |
| (2) | Ethanol | 8 |
| (3) | Glycerin | 1 |
| (4) | 1,3-Butylene glycol | 5 |
| (5) | POE (12) dimethylpolysiloxane (SH3773M, manufactured by Dow Corning Silicone) | 1.3 |
| (6) | Polyoxyethylene alkyl (12-15) ether phosphate (2E.O.) (DDP-2, manufactured by Nikko Chemicals Co., Ltd.) | 0.01 |
| (7) | Tripropylene glycol dipivalate | 0.1 |
| (8) | Tranexamic acid methylamide hydrochloride | 1 |
| (9) | Citric acid | 0.02 |
| (10) | Sodium citrate | 0.08 |
| (11) | Sodium hexametaphosphate | 0.03 |
| (12) | Phenoxyethanol | 0.3 |
| (13) | Fragrance | Appropriate amount |

### (Production method)

To a water phase prepared by mixing components (3), (4) and (8) to (11) with component (1), a mixture prepared by mixing and dissolving components (5), (7), (12) and (13) to component (2) and finally component (6) was added, mixed and dissolved to obtain a lotion.

The obtained lotion showed a turbidity change of 5% at 50°C and -10°C and showed excellent appearance stability (⊚).

### Formulation Example 3 Beauty Lotion

| (Component) | | (mass%) |
|---|---|---|
| (1) | Purified water | Balance |
| (2) | Glycerin | 5 |
| (3) | Dipropylene glycol | 7 |
| (4) | 1,3-Butylene glycol | 3 |
| (5) | PEG-20 | 2 |
| (6) | Carbomer | 0.2 |
| (7) | Caustic potash | 0.05 |
| (8) | POE (12) dimethylpolysiloxane (SH3773M, manufactured by Dow Coming Silicone) | 2 |
| (9) | Potassium stearoyl glutamate | 0.1 |
| (10) | Isostearic acid | 0.15 |
| (11) | Tranexamic acid | 1 |
| (12) | EDTA-3Na·2H₂O | 0.03 |
| (13) | Phenoxyethanol | 0.3 |
| (14) | Methylparaben | 0.15 |
| (15) | Fragrance | Appropriate amount |

### (Production method)

To a water phase prepared by mixing components (2), (4), (5), (6), (11), (12) and (15) with component (1) and being neutralized with component (7), a mixture prepared by mixing and dissolving components (8), (10) and (13) to (15) to component (3) and finally component (9) was added, mixed and dissolved to obtain a beauty lotion.

The obtained beauty lotion showed a turbidity change of 5% at 50°C and -10°C and showed excellent appearance stability (⊚).

### Formulation Example 4 Beauty Lotion mask

| (Component) | | (mass%) |
|---|---|---|
| (1) | Purified water | Balance |
| (2) | Ethanol | 3 |
| (3) | Glycerin | 5 |
| (4) | Dipropylene glycol | 5 |
| (5) | Xylitol | 3 |
| (6) | Sorbitol | 2 |
| (7) | Xanthan gum | 0.18 |
| (8) | POE (12) dimethylpolysiloxane (SH3773M, manufactured by Dow Corning Silicone) | 2 |
| (9) | Potassium cocoyl glutamate | 0.1 |
| (10) | Diphenylsiloxyphenyltrimethicone | 0.15 |
| (11) | Ascorbic acid glucoside | 1 |
| (12) | EDTA-3Na·2H₂O | 0.03 |
| (13) | Citric acid | 0.03 |
| (14) | Sodium citrate | 0.07 |
| (15) | Phenoxyethanol | 0.3 |
| (16) | Methylparaben | 0.1 |
| (17) | Fragrance | Appropriate amount |

### (Production method)

To a water phase prepared by mixing components (3) to (7) and (11) to (14) with component (1), a mixture prepared by mixing and dissolving components (8), (10) and (15) to (17) to component (2) and finally component (9) was added, mixed and dissolved to obtain a beauty lotion to obtain a mask impregnated with the lotion.

The obtained beauty lotion showed a turbidity change of 5% at 50°C and -10°C and showed excellent appearance stability (⊚).

In accordance with the formulations and methodes shown in the following "Table 4" to "Table 8", cosmetics containing a vesicle-containing composition were produced and the smell stability thereof was evaluated. A method for evaluating smell stability is as follows.

### Smell stability

The Smell stability was evaluated by a specialist based on smell from a bottle mouth of a sample tube with respect to a reference product (stored at room temperature 20°C, for a month) and a stored product at 50°C for a month.

Note that, evaluation criteria are as follows.
⊚: No change in smell relative to the reference product.
○: No substantial change in smell relative to the reference product.
Δ: A slight change in smell relative to the reference product is sensed.
×: Unacceptable change in smell as cosmetics relative to the reference product is sensed.

### "Lotion"

**Table 4**

| | Component | Example 1 | Example 2 | Example 3 | Example 4 | Example 5 | Example 6 | Comparative Example 1 | Comparative Example 2 |
|---|---|---|---|---|---|---|---|---|---|
| **1** | Purified water | Balance | Balance | Balance | Balance | Balance | Balance | Balance | Balance |
| **2** | Ethanol | **3** | **5** | **5** | **5** | **5** | **5** | **5** | **5** |
| **3** | Glycerin | **3** | **3** | **3** | **3** | **3** | **3** | **3** | **3** |
| **4** | 1,3-butylene glycol | **5** | **5** | **5** | **5** | **5** | **5** | **5** | **5** |
| **5** | POE (12) dimethylpolysiloxane *1 | **0.5** | **0.5** | **0.5** | **0.5** | **0.5** | **0.5** | **0.5** | **0.5** |
| **6** | Sodium stearoyl methyl taurate | **0.02** | **0.02** | **0.02** | **0.02** | **0.02** | **0.02** | **0.02** | **0.02** |
| **7** | Pentaerythrityl tetra-2-ethylhexanoate | **0.05** | **0.05** | **0.05** | **0.05** | **0.05** | **0.05** | **0.05** | **0.05** |
| **8** | Citric acid | **0.03** | **0.03** | **0.03** | **0.03** | **0.03** | **0.03** | **0.03** | **0.03** |
| **9** | Sodium citrate | **0.07** | **0.07** | **0.07** | **0.07** | **0.07** | **0.07** | **0.07** | **0.07** |
| **10** | **EDTA-2Na·2H₂O** | **0.03** | **0.03** | **0.03** | **0.03** | **0.03** | **0.03** | **0.03** | **0.03** |
| **11** | Sodium pyrosulphite | **0.001** | **0.003** | **0.01** | **0.03** | **-** | **0.05** | **0.1** | **-** |
| **12** | Dibutylhydroxytoluene | **-** | **-** | **-** | **-** | **0.01** | **-** | **-** | **-** |
| **13** | Phenoxyethanol | Appropriate amount | Appropriate amount | Appropriate amount | Appropriate amount | Appropriate amount | Appropriate amount | Appropriate amount | Appropriate amount |
| | Smell stability **(50°C)** | ○ | ⊚ | ⊚ | ○ | ○ | Δ(*2) | × | × |

| | | | | | | | | | |
|---|---|---|---|---|---|---|---|---|---|
| *1: SH3773M manufactured by Dow Corning Toray Co., Ltd. *2: A change in smell of polyether modified silicone was not shown, but a change in smell of pyrosulfite was shown. | | | | | | | | | |

### (Examples 1 to 6 are not according to the invention)

### (Production method)

To a water phase prepared by mixing and dissolving components 3, 4, 8, 9, 10 and 11 to component 1, a mixture prepared by mixing and dissolving components 5, 7, 12 and 13 to component 2 was added. To this, finally a mixture of component 6 in water and heated to dissolve was added and mixed and dissolved to obtain a lotion.

From the "Table 4", it is found that Examples of the present invention in which sodium pyrosulphite or dibutylhydroxytoluene was blended have extremely excellent smell stability.

### "Lotion"

**Table 5**

| | Component | Example 7 |
|---|---|---|
| **1** | Purified water | Balance |
| **2** | Ethanol | **3** |
| **3** | Glycerin | **8** |
| **4** | 1,3-butylene glycol | **7** |
| **5** | PEG/PPG-14/7 dimethyl ether | **3** |
| **6** | POE(12)dimethylpolysiloxane | **1** |
| **7** | Polyoxyethylene alkyl (12 to 15) ether phosphate (2 E.O.) | **0.05** |
| **8** | Tripropylene glycol dipivalate | **0.05** |
| **9** | Potassium 4-methoxysalicylate | **1** |
| **10** | Citric acid | **0.03** |
| **11** | Sodium citrate | **0.07** |
| **12** | **EDTA-2Na·2H₂O** | **0.03** |
| **13** | Sodium pyrosulphite | **0.005** |
| **14** | Phenoxyethanol | Appropriate amount |
| **15** | Fragrance | Appropriate amount |
| | Smell stability **(50°C)** | ⊚ |

| | | |
|---|---|---|
| *1: SH3773M manufactured by Dow Corning Toray Co., Ltd. | | |

### (Production method)

To a water phase prepared by mixing and dissolving components 3, 4, 9, 10, 11, 12 and 13 to component 1, a mixture prepared by mixing and dissolving components 5, 6, 8, 14 and 15 to component 2 was added. To this, finally a mixture of component 7 was added and mixed and dissolved to obtain a lotion.

### "Lotion"

**Table 6**

| | Component | Example 8 |
|---|---|---|
| **1** | Purified water | Balance |
| **2** | Ethanol | **8** |
| **3** | Glycerin | **3** |
| **4** | Dipropylene glycol | **3** |
| **5** | POE(12)dimethylpolysiloxane *1 | **0.5** |
| **6** | Polyoxyethylene alkyl (12 to 15) ether phosphate (2 E.O.) | **0.01** |
| **7** | Glyceryl tri-2-ethylhexanoate | **0.1** |
| **8** | Tranexamic acid methyl amide hydrochloride | **1** |
| **9** | Lactic acid | **0.002** |
| **10** | Sodium lactate solution (50%) | **0.018** |
| **11** | Sodium hexametaphosphate | **0.03** |
| **12** | Dibutylhydroxytoluene | **0.01** |
| **13** | Phenoxyethanol | Appropriate amount |
| **14** | Fragrance | Appropriate amount |
| | Smell stability **(50°C)** | ○ |

| | | |
|---|---|---|
| *1: SH3773M manufactured by Dow Corning Toray Co., Ltd. | | |

### (Production method)

To a water phase prepared by mixing and dissolving components 3, 4, 8, 9, 10, 11 and 12 to component 1, a mixture prepared by mixing and dissolving components 5, 7, 13 and 14 to component 2 was added. To this, finally a mixture of component 6 was added and mixed and dissolved to obtain a lotion.

### "Beauty lotion"

**Table 7**

| | Component | Example 9 |
|---|---|---|
| **1** | Purified water | Balance |
| **2** | Glycerin | **5** |
| **3** | Dipropylene glycol | **3** |
| **4** | 1,3-butylene glycol | **7** |
| **5** | PEG-20 | **2** |
| **6** | Carbomer | **0.1** |
| **7** | Caustic potash | **0.05** |
| **8** | POE(12)dimethylpolysiloxane *1 | **1.3** |
| **9** | Sodium stearoyl methyl taurate | **0.05** |
| **10** | Isostearic acid | **0.15** |
| **11** | Tranexamic acid | **1** |
| **12** | **EDTA-3Na·2H₂O** | **0.03** |
| **13** | Sodium pyrosulfite | **0.003** |
| **14** | Phenoxyethanol | Appropriate amount |
| **15** | Methylparaben | Appropriate amount |
| **16** | Fragrance | Appropriate amount |
| | Smell stability (50°C) | ⊚ |

| | | |
|---|---|---|
| *1: SH3773M manufactured by Dow Corning Toray Co., Ltd. | | |

### (Production method)

To a water phase prepared by mixing and dissolving components 2, 4, 5, 6, 11, 12 and 13 to component 1 and being neutralized with component 7, a mixture prepared by mixing and dissolving components 8, 10, 14, 15 and 16 to component 3 was added. To this, finally a mixture of component 9 was added and mixed and dissolved to obtain a beauty lotion.

### "Mask impregnated with beauty lotion"

**Table 8**

| | Component | Example 10 |
|---|---|---|
| **1** | Purified water | Balance |
| **2** | Ethanol | 3 |
| **3** | Glycerin | **1** |
| **4** | Dipropylene glycol | **5** |
| **5** | Xylitol | **3** |
| **6** | Sorbitol | **5** |
| **7** | Xanthane gum | **0.18** |
| **8** | POE(12)dimethylpolysiloxane *1 | **1.8** |
| **9** | Potassium cocoyl glutamate | **0.1** |
| **10** | Diphenylsiloxy phenyl trimethicone | **0.15** |
| **11** | Ascorbic acid glucoside | **2** |
| **12** | **EDTA-3Na·2H₂O** | **0.03** |
| **13** | Citric acid | **0.03** |
| **14** | Sodium citrate | **0.07** |
| **15** | Sodium pyrosulfite | **0.015** |
| **16** | Phenoxyethanol | Appropriate amount |
| **17** | Methylparaben | Appropriate amount |
| **18** | Fragrance | Appropriate amount |
| | Smell stability **(50°C)** | ⊚ |

| | | |
|---|---|---|
| *1: SH3773M, manufactured by Dow Coming Silicone | | |

### (Production method)

To a water phase prepared by mixing and dissolving components 3, 4, 5, 6, 7, 11, 12, 13, 14 and 15 with component 1 to dissolve them, a mixture prepared by mixing components 8, 10, 16, 17, 18 with component 2 to dissolve them was added. Finally, component 9 was added and to the resulting mixture to mix and dissolve it. Non-woven cloth was impregnated with the obtained solution to obtain a mask.

From the results of the "Table 5" to "Table 8", it is found that Examples of the present invention in which sodium pyrosulphite or dibutylhydroxytoluene was blended have extremely excellent smell stability.

## Claims

1. A vesicle-containing composition comprising:
(A) a silicone-based surfactant,
(B) one or more anionic surfactants selected from polyoxyethylene alkyl (12 to 15) ether phosphate, acyl methyl taurate and acyl glutamate in an amount of 0.001 to 0.2 mass%,
(C) a polar oil having IOB of 0.05 to 0.80, and/or, silicone oil, and
(D) water containing a water-soluble medicinal agent selected from tranexamic acid, tranexamic acid methyl amide hydrochloride, potassium 4-methoxysalicylate, ascorbic acid glucoside and ethyl vitamin C in an amount of 0.5 to 5 mass% based on the composition,
wherein the (A) silicone-based surfactant forms vesicles; the (B) anionic surfactant(s) attaches to a surface of the vesicles; and the (C) polar oil and/or silicone oil is present within a bilayer membrane of the vesicles,
wherein acyl methyl taurate is selected from coconut oil fatty acid methyl taurate, palm kernel oil fatty acid methyl taurate, hydrogenated palm kernel oil fatty acid methyl taurate, beef tallow fatty acid methyl taurate, hydrogenated beef tallow fatty acid methyl taurate, caproyl methyl taurate, lauroyl methyl taurate, myristoyl methyl taurate, palmitoyl methyl taurate, stearoyl methyl taurate, oleoyl methyl taurate and cocoyl methyl taurate,
wherein acyl glutamate is selected from stearoyl glutamate, cocoyl glutamate, coconut oil fatty acid glutamate, lauroyl glutamate, palm kernel oil fatty acid glutamate, hydrogenated palm kernel oil fatty acid glutamate, beef tallow fatty acid glutamate and hydrogenated beef tallow fatty acid glutamate, and
wherein a counter ion to the salts is selected from Na, K, triethanol amine and ammonia.

2. The vesicle-containing composition according to Claim 1, wherein the (A) silicone-based surfactant is polyoxyalkylene-modified silicone represented by the following formula (1): 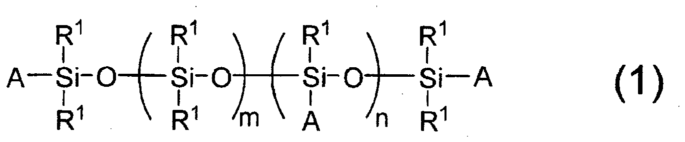 wherein R¹ represents hydrogen or an alkyl group having 1 to 6 carbon atoms; at least one of A is a polyoxyalkylene group represented by the formula: -(CH₂)ₐ-(C₂H₄O)_{b}-(C₃H₆O)_{c}-R² wherein R² represents hydrogen or an alkyl group having 1 to 6 carbon atoms, a represents an integer of 1 to 6, b represents an integer of 0 to 50, c represents an integer of 0 to 50 and b + c represents at least 5; the other(s) of A represents hydrogen or an alkyl group having 1 to 6 carbon atoms; m represents an integer of 1 to 200; and n represents an integer of 0 to 50.

3. The vesicle-containing composition according to Claim 1 or 2, wherein a content of the (C) polar oil having IOB of 0.05 to 0.80, and/or, silicone oil is 0.001 to 0.3 mass%.

4. The vesicle-containing composition according to any of Claims 1 to 3, wherein a vesicle formed of the (A) silicone-based surfactant further contains, (E) sodium pyrosulfite in an amount of 0.001 to 0.05 mass% and/or dibutylhydroxytoluene in an amount of 0.005 to 0.05 mass%.

5. The vesicle-containing composition according to any of Claims 1 to 4, wherein the (D) water containing a water-soluble medicinal agent in an amount of 0.5 to 5 mass% based on the composition further contains one or two or more selected from the group consisting of ethanol, propylene glycol, dipropylene glycol and 1,3-butylene glycol.

6. A method for producing a vesicle-containing composition, comprising:
mixing (A) a silicone-based surfactant and (C) a polar oil having IOB of 0.05 to 0.80, and/or, silicone oil,
mixing with (D) water containing a water-soluble medicinal agent selected from the group consisting of tranexamic acid, tranexamic acid methyl amide hydrochloride, potassium 4-methoxysalicylate, ascorbic acid glucoside or ethyl vitamin C, and
adding and mixing (B) one or more anionic surfactants selected from polyoxyethylenealkyl (12 to 15) ether phosphate, acyl methyl taurate and acyl glutamate,
wherein acyl methyl taurate is selected from coconut oil fatty acid methyl taurate, palm kernel oil fatty acid methyl taurate, hydrogenated palm kernel oil fatty acid methyl taurate, beef tallow fatty acid methyl taurate, hydrogenated beef tallow fatty acid methyl taurate, caproyl methyl taurate, lauroyl methyl taurate, myristoyl methyl taurate, palmitoyl methyl taurate, stearoyl methyl taurate, oleoyl methyl taurate and cocoyl methyl taurate,
wherein acyl glutamate is selected from stearoyl glutamate, cocoyl glutamate, coconut oil fatty acid glutamate, lauroyl glutamate, palm kernel oil fatty acid glutamate, hydrogenated palm kernel oil fatty acid glutamate, beef tallow fatty acid glutamate and hydrogenated beef tallow fatty acid glutamate, and
wherein a counter ion to the salts is selected from Na, K, triethanol amine and ammonia.

7. The method for producing a vesicle-containing composition according to Claim 6, comprising:
mixing the (A) silicone-based surfactant and (C) a polar oil having IOB of 0.05 to 0.80, and/or, silicone oil,
mixing with a mixture obtained by mixing (E) sodium pyrosulphite and/or dibutylhydroxytoluene and (D) water containing a water-soluble medicinal agent selected from the group consisting of tranexamic acid, tranexamic acid methyl amide hydrochloride, potassium 4-methoxysalicylate, ascorbic acid glucoside or ethyl vitamin C, and
adding and mixing one or more anionic surfactant (B) selected from polyoxyethylenealkyl (12 to 15) ether phosphate, acyl methyl taurate, and acyl glutamate,
wherein acyl methyl taurate is selected from coconut oil fatty acid methyl taurate, palm kernel oil fatty acid methyl taurate, hydrogenated palm kernel oil fatty acid methyl taurate, beef tallow fatty acid methyl taurate, hydrogenated beef tallow fatty acid methyl taurate, caproyl methyl taurate, lauroyl methyl taurate, myristoyl methyl taurate, palmitoyl methyl taurate, stearoyl methyl taurate, oleoyl methyl taurate and cocoyl methyl taurate,
wherein acyl glutamate is selected from stearoyl glutamate, cocoyl glutamate, coconut oil fatty acid glutamate, lauroyl glutamate, palm kernel oil fatty acid glutamate, hydrogenated palm kernel oil fatty acid glutamate, beef tallow fatty acid glutamate and hydrogenated beef tallow fatty acid glutamate, and
wherein a counter ion to the salts is selected from Na, K, triethanol amine and ammonia.

## Patentansprüche

1. Vesikelhaltige Zusammensetzung, umfassend:
(A) ein silikonbasiertes oberflächenaktives Mittel,
(B) ein oder mehrere anionische oberflächenaktive Mittel ausgewählt aus Polyoxyethylenalkyl(12 bis 15) etherphosphat, Acylmethyltaurat und Acylglutamat in einer Menge von 0.001 bis 0.2 Masse%,
(C) ein polares Öl mit einem IOB von 0.05 bis 0.80 und/oder Silikonöl, und
(D) Wasser, welches ein wasserlösliches Arzneimittel ausgewählt aus Tranexamsäure, Tranexamsäuremethylamid-Hydrochlorid, Kalium-4-methoxysalicylat, Ascorbinsäureglukosid und Ethyl-Vitamin C in einer Menge von 0.5 bis 5 Masse%, bezogen auf die Zusammensetzung, enthält,
wobei das silikonbasierte oberflächenaktive Mittel (A) Vesikel bildet; das/die anionische(n) oberflächenaktive(n) Mittel (B) an eine Oberfläche der Vesikel bindet/binden; und das polare Öl und/oder Silikonöl (C) in einer Doppelschichtmembran der Vesikel vorliegt,
wobei das Acylmethyltaurat aus Kokosnussölfettsäuremethyltaurat, Palmkernölfettsäuremethyltaurat, hydriertem Palmkernölfettsäuremethyltaurat, Rindertalgfettsäuremethyltaurat, hydriertem Rindertalgfettsäuremethyltaurat, Caproylmethyltaurat, Lauroylmethyltaurat, Myristoylmethyltaurat, Palmitoylmethyltaurat, Stearoylmethyltaurat, Oleoylmethyltaurat und Cocoylmethyltaurat ausgewählt ist,
wobei das Acylglutamat aus Stearoylglutamat, Cocoylglutamat, Kokosnussölfettsäureglutamat, Lauroylglutamat, Palmkernölfettsäureglutamat, hydriertem Palmkernölfettsäureglutamat, Rindertalgfettsäureglutamat und hydriertem Rindertalgfettsäureglutamat ausgewählt ist, und
wobei ein Gegenion der Salze aus Na, K, Triethanolamin und Ammoniak ausgewählt ist.

2. Vesikelhaltige Zusammensetzung nach Anspruch 1, wobei das silikonbasierte oberflächenaktive Mittel (A) ein durch die nachfolgende Formel (1) dargestelltes Polyoxyalkylenmodifiziertes Silikon ist: 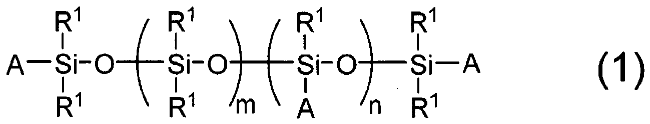 wobei R¹ für Wasserstoff oder eine Alkylgruppe mit 1 bis 6 Kohlenstoffatomen steht; zumindest eines von A für eine durch die Formel: -(CH₂)ₐ-(C₂H₄O)_{b}-(C₃H₆O)_{c}-R² dargestellte Polyoxyalkylengruppe steht, worin R² für Wasserstoff oder eine Alkylgruppe mit 1 bis 6 Kohlenstoffatomen steht, a für eine ganze Zahl von 1 bis 6 steht, b für eine ganze Zahl von 0 bis 50 steht, c für eine ganze Zahl von 0 bis 50 steht, und b + c für zumindest 5 stehen; die anderen A's für Wasserstoff oder eine Alkylgruppe mit 1 bis 6 Kohlenstoffatomen stehen; m für eine ganze Zahl von 1 bis 200 steht; und n für eine ganze Zahl von 0 bis 50 steht.

3. Vesikelhaltige Zusammensetzung nach Anspruch 1 oder 2, wobei der Gehalt des polaren Öls mit einem IOB von 0.05 bis 0.80 und/oder Silikonöls (C) 0.001 bis 0.3 Masse% beträgt.

4. Vesikelhaltige Zusammensetzung nach einem der Ansprüche 1 bis 3, wobei ein aus dem silikonbasierten oberflächenaktiven Mittel (A) gebildetes Vesikel weiterhin (E) Natriumpyrosulfit in einer Menge von 0.001 bis 0.05 Masse% und/oder Dibutylhydroxytoluol in einer Menge von 0.005 bis 0.05 Masse% enthält.

5. Vesikelhaltige Zusammensetzung nach einem der Ansprüche 1 bis 4, wobei das Wasser (D), welches ein wasserlösliches Arzneimittel in einer Menge von 0.5 bis 5 Masse%, bezogen auf die Zusammensetzung, enthält, weiterhin eines oder zwei oder mehrere ausgewählt aus der Gruppe bestehend aus Ethanol, Propylenglykol, Dipropylenglykol und 1,3-Butylenglykol enthält.

6. Verfahren zur Herstellung einer vesikelhaltigen Zusammensetzung, umfassend:
Mischen von (A) einem silikonbasierten oberflächenaktiven Mittel und (C) einem polaren Öl mit einem IOB von 0.05 bis 0.80 und/oder Silikonöl,
Mischen mit (D) Wasser, welches ein wasserlösliches Arzneimittel ausgewählt aus der Gruppe bestehend aus Tranexamsäure, Tranexamsäuremethylamid-Hydrochlorid, Kalium-4-methoxysalicylat, Ascorbinsäureglukosid oder Ethyl-Vitamin C enthält, und
Hinzufügen und Einmischen von (B) einem oder mehreren anionischen oberflächenaktiven Mitteln ausgewählt aus Polyoxyethylenalkyl(12 bis 15)etherphosphat, Acylmethyltaurat und Acylglutamat,
wobei das Acylmethyltaurat aus Kokosnussölfettsäuremethyltaurat, Palmkernölfettsäuremethyltaurat, hydriertem Palmkernölfettsäuremethyltaurat, Rindertalgfettsäuremethyltaurat, hydriertem Rindertalgfettsäuremethyltaurat, Caproylmethyltaurat, Lauroylmethyltaurat, Myristoylmethyltaurat, Palmitoylmethyltaurat, Stearoylmethyltaurat, Oleoylmethyltaurat und Cocoylmethyltaurat ausgewählt ist,
wobei das Acylglutamat aus Stearoylglutamat, Cocoylglutamat, Kokosnussölfettsäureglutamat, Lauroylglutamat, Palmkernölfettsäureglutamat, hydriertem Palmkernölfettsäureglutamat, Rindertalgfettsäureglutamat und hydriertem Rindertalgfettsäureglutamat ausgewählt ist, und
wobei ein Gegenion der Salze aus Na, K, Triethanolamin und Ammoniak ausgewählt ist.

7. Verfahren zur Herstellung einer vesikelhaltigen Zusammensetzung nach Anspruch 6, umfassend:
Mischen von (A) dem silikonbasierten oberflächenaktiven Mittel und (C) einem polaren Öl mit einem IOB von 0.05 bis 0.80 und/oder Silikonöl,
Mischen mit einem Gemisch, das durch Mischen von (E) Natriumpyrosulfit und/oder Dibutylhydroxytoluol und (D) Wasser, welches ein wasserlösliches Arzneimittel ausgewählt aus der Gruppe bestehend aus Tranexamsäure, Tranexamsäuremethylamid-Hydrochlorid, Kalium-4-methoxysalicylat, Ascorbinsäureglukosid oder Ethyl-Vitamin C enthält, erhalten worden ist, und
Hinzufügen und Einmischen von (B) einem oder mehreren anionischen oberflächenaktiven Mitteln ausgewählt aus Polyoxyethylenalkyl(12 bis 15)etherphosphat, Acylmethyltaurat und Acylglutamat,
wobei das Acylmethyltaurat aus Kokosnussölfettsäuremethyltaurat, Palmkernölfettsäuremethyltaurat, hydriertem Palmkernölfettsäuremethyltaurat, Rindertalgfettsäuremethyltaurat, hydriertem Rindertalgfettsäuremethyltaurat, Caproylmethyltaurat, Lauroylmethyltaurat, Myristoylmethyltaurat, Palmitoylmethyltaurat, Stearoylmethyltaurat, Oleoylmethyltaurat und Cocoylmethyltaurat ausgewählt ist,
wobei das Acylglutamat aus Stearoylglutamat, Cocoylglutamat, Kokosnussölfettsäureglutamat, Lauroylglutamat, Palmkernölfettsäureglutamat, hydriertem Palmkernölfettsäureglutamat, Rindertalgfettsäureglutamat und hydriertem Rindertalgfettsäureglutamat ausgewählt ist, und
wobei ein Gegenion der Salze aus Na, K, Triethanolamin und Ammoniak ausgewählt ist.

## Revendications

1. Composition contenant des vésicules, comprenant :
(A) un tensioactif à base de silicone,
(B) un ou plusieurs tensioactifs anioniques choisis parmi un éther phosphate de polyoxyéthylènealkyle (12 à 15), un acyl méthyl taurate et un glutamate d'acyle en une quantité de 0,001 à 0,2 % en masse,
(C) une huile polaire ayant une balance inorganique/organique (IOB) de 0,05 à 0,80, et/ou une huile de silicone, et
(D) de l'eau contenant un agent médicinal hydrosoluble choisi parmi l'acide tranexamique, le chlorhydrate de méthyl amide d'acide tranexamique, le 4-méthoxysalicylate de potassium, le glucoside d'acide ascorbique et l'éthyl vitamine C en une quantité de 0,5 à 5 % en masse par rapport à la composition,
dans laquelle (A) le tensioactif à base de silicone forme des vésicules ; (B) le(s) tensioactif(s) anionique(s) s'attache(nt) à une surface des vésicules ; et (C) l'huile polaire et/ou huile de silicone est présente à l'intérieur d'une membrane bicouche des vésicules,
dans laquelle l'acyl méthyl taurate est choisi parmi un méthyl taurate d'acide gras d'huile de coco, un méthyl taurate d'acide gras d'huile de palmiste, un méthyl taurate d'acide gras d'huile de palmiste hydrogénée, un méthyl taurate d'acide gras de suif de boeuf, un méthyl taurate d'acide gras de suif de boeuf hydrogéné, un caproyl méthyl taurate, un lauroyl méthyl taurate, un myristoyl méthyl taurate, un palmitoyl méthyl taurate, un stéaroyl méthyl taurate, un oléoyl méthyl taurate et un cocoyl méthyl taurate,
dans laquelle le glutamate d'acyle est choisi parmi un glutamate de stéaroyle, un glutamate de cocoyle, un glutamate d'acide gras d'huile de coco, un glutamate de lauroyle, un glutamate d'acide gras d'huile de palmiste, un glutamate d'acide gras d'huile de palmiste hydrogénée, un glutamate d'acide gras de suif de boeuf et un glutamate d'acide gras de suif de boeuf hydrogéné, et
dans laquelle un contre-ion des sels est choisi parmi Na, K, la triéthanolamine et l'ammoniaque.

2. Composition contenant des vésicules selon la revendication 1, dans laquelle (A) le tensioactif à base de silicone est une silicone modifiée par un polyoxyalkylène représentée par la formule (1) suivante : 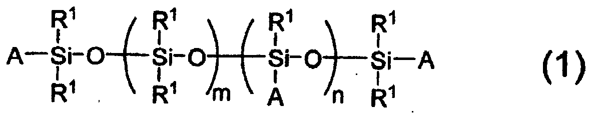 dans laquelle R¹ représente un hydrogène ou un groupe alkyle ayant 1 à 6 atomes de carbone ; au moins l'un des A est un groupe polyoxyalkylène représenté par la formule : -(CH₂)ₐ-(C₂H₄O)_{b}-(C₃H₆O)_{c}-R² où R² représente un hydrogène ou un groupe alkyle ayant 1 à 6 atomes de carbone, a représente un entier de 1 à 6, b représente un entier de 0 à 50, c représente un entier de 0 à 50 et b+c représente au moins 5 ; le(s) autre(s) A représente(nt) un hydrogène ou un groupe alkyle ayant 1 à 6 atomes de carbone ; m représente un entier de 1 à 200 ; et n représente un entier de 0 à 50.

3. Composition contenant des vésicules selon la revendication 1 ou 2, dans laquelle une teneur en (C) l'huile polaire ayant une IOB de 0,05 à 0,80 et/ou en l'huile de silicone est de 0,001 à 0,3 % en masse.

4. Composition contenant des vésicules selon l'une quelconque des revendications 1 à 3, dans laquelle une vésicule formée de (A) le tensioactif à base de silicone contient en outre (E) du pyrosulfite de sodium en une quantité de 0,001 à 0,05 % en masse et/ou du dibutylhydroxytoluène en une quantité de 0,005 à 0,05 % en masse.

5. Composition contenant des vésicules selon l'une quelconque des revendications 1 à 4, dans laquelle (D) l'eau contenant un agent médicinal hydrosoluble en une quantité de 0,5 à 5 % en masse par rapport à la composition contient en outre un ou deux ingrédients ou plus choisis dans le groupe constitué par l'éthanol, le propylèneglycol, le dipropylèneglycol et le 1,3-butylèneglycol.

6. Procédé pour produire une composition contenant des vésicules, comprenant :
le mélange de (A) un tensioactif à base de silicone et de (C) une huile polaire ayant une IOB de 0,05 à 0,80 et/ou d'une huile de silicone,
le mélange avec (D) de l'eau contenant un agent médicinal hydrosoluble choisi dans le groupe constitué par l'acide tranexamique, le chlorhydrate de méthyl amide d'acide tranexamique, le 4-méthoxysalicylate de potassium, le glucoside d'acide ascorbique ou l'éthyl vitamine C, et
l'addition et le mélange de (B) un ou plusieurs tensioactifs anioniques choisis parmi un éther phosphate de polyoxyéthylènealkyle (12 à 15), un acyl méthyl taurate et un glutamate d'acyle,
dans lequel l'acyl méthyl taurate est choisi parmi un méthyl taurate d'acide gras d'huile de coco, un méthyl taurate d'acide gras d'huile de palmiste, un méthyl taurate d'acide gras d'huile de palmiste hydrogénée, un méthyl taurate d'acide gras de suif de boeuf, un méthyl taurate d'acide gras de suif de boeuf hydrogéné, un caproyl méthyl taurate, un lauroyl méthyl taurate, un myristoyl méthyl taurate, un palmitoyl méthyl taurate, un stéaroyl méthyl taurate, un oléoyl méthyl taurate et un cocoyl méthyl taurate,
dans lequel le glutamate d'acyle est choisi parmi un glutamate de stéaroyle, un glutamate de cocoyle, un glutamate d'acide gras d'huile de coco, un glutamate de lauroyle, un glutamate d'acide gras d'huile de palmiste, un glutamate d'acide gras d'huile de palmiste hydrogénée, un glutamate d'acide gras de suif de boeuf et un glutamate d'acide gras de suif de boeuf hydrogéné, et
dans lequel un contre-ion des sels est choisi parmi Na, K, la triéthanolamine et l'ammoniaque.

7. Procédé pour produire une composition contenant des vésicules selon la revendication 6, comprenant :
le mélange de (A) un tensioactif à base de silicone et de (C) une huile polaire ayant une IOB de 0,05 à 0,80 et/ou d'une huile de silicone,
le mélange avec un mélange obtenu en mélangeant (E) du pyrosulfite de sodium et/ou du dibutylhydroxytoluène et (D) de l'eau contenant un agent médicinal hydrosoluble choisi dans le groupe constitué par l'acide tranexamique, le chlorhydrate de méthyl amide d'acide tranexamique, le 4-méthoxysalicylate de potassium, le glucoside d'acide ascorbique ou l'éthyl vitamine C, et
l'addition et le mélange d'un ou plusieurs tensioactifs anioniques (B) choisis parmi un éther phosphate de polyoxyéthylènealkyle (12 à 15), un acyl méthyl taurate et un glutamate d'acyle,
dans lequel l'acyl méthyl taurate est choisi parmi un méthyl taurate d'acide gras d'huile de coco, un méthyl taurate d'acide gras d'huile de palmiste, un méthyl taurate d'acide gras d'huile de palmiste hydrogénée, un méthyl taurate d'acide gras de suif de boeuf, un méthyl taurate d'acide gras de suif de boeuf hydrogéné, un caproyl méthyl taurate, un lauroyl méthyl taurate, un myristoyl méthyl taurate, un palmitoyl méthyl taurate, un stéaroyl méthyl taurate, un oléoyl méthyl taurate et un cocoyl méthyl taurate,
dans lequel l'acyl glutamate est choisi parmi un glutamate de stéaroyle, un glutamate de cocoyle, un glutamate d'acide gras d'huile de coco, un glutamate de lauroyle, un glutamate d'acide gras d'huile de palmiste, un glutamate d'acide gras d'huile de palmiste hydrogénée, un glutamate d'acide gras de suif de boeuf et un glutamate d'acide gras de suif de boeuf hydrogéné, et
dans lequel un contre-ion des sels est choisi parmi Na, K, la triéthanolamine et l'ammoniaque.
